# EUROPEAN PATENT SPECIFICATION

(11) **EP 3 678 632 B1**
(45) Date of publication and mention of the grant of the patent: **06.09.2023**
(21) Application number: 18854183.3
(22) Date of filing: 29.08.2018
(51) Int. Cl.: A61K 8/11, A61K 8/73, A61K 8/92, A61Q 1/02, A61Q 19/00

(54) **A TRUFFLE CONFIGURED COSMETIC ARTICLE**
MIT TRÜFFEL KONFIGURIERTER KOSMETIKARTIKEL
ARTICLE COSMÉTIQUE CONFIGURÉ EN TRUFFE

(30) Priority: 05.09.2017 US 201715695661
(43) Date of publication of application: 15.07.2020
(73) Proprietor: REA Innovations, Inc., Seattle, Washington 98117 (US)
(72) Inventor: AKRIDGE, Robb, Seattle, Washington 98117 (US)
(74) Representative: HGF
(86) International application number: PCT/US2018/048575
(87) International publication number: WO 2019/050742

(56) References cited:
- JP-A- 2014 037 397
- US-A1- 2006 147 390
- US-A1- 2007 172 565
- US-A1- 2008 089 913
- US-A1- 2009 181 254
- US-A1- 2011 052 680
- US-A1- 2011 223 224
- US-A1- 2017 042 774
- Anonymous: "Fleet 185B Liquid Glycerin Suppositories, 4 ea. : Amazon.co.uk: Health & Personal Care", , 1 March 2016 (2016-03-01), XP055824679, Retrieved from the Internet: URL:https://www.amazon.co.uk/Fleet-185B-Li quid-Glycerin-Suppositories/dp/B0000530H5 [retrieved on 2021-07-15]
- Erwachsene Für: "Gebrauchsinformation: Information für den Anwender", , 1 August 2014 (2014-08-01), XP55824987, Retrieved from the Internet: URL:https://image.wub-service.de/resources /static/des/210715/44/02/44023.pdf [retrieved on 2021-07-16]
- EdensSecret1: "How to decorate bath bombs", EDENSSECRET1, 15 June 2016 (2016-06-15), XP054979310, Retrieved from the Internet: URL:https://www.youtube.com/watch?v=E2rrgI 2wbWs [retrieved on 2018-10-02]

## Description

### Technical Field

This invention relates generally to cosmetics and more particularly to a method for preparing a skin formulation.

### Background of the Invention

Typical high end or signature cosmetic departments will display multiple brands, each with their own signature look and appeal. Each cosmetic brand typically has a glass counter, some with stools or other seating arrangements. On top of the retail counter, in glass cases and behind the counter typically are arranged bottles, tubes and jars according to the cosmetic purpose, including, for example, anti-aging moisturizers or skin conditioners, and several different cosmetic formulations. In each arrangement or brand, there are a large number of different containers; accordingly, there is typically a beauty advisor to assist and to sell to the individual customers. The beauty advisor will, after consulting with the customer, typically advise a skin care routine of three basic steps, to cleanse, treat and protect. For each step, there are numerous bottles and jars containing specialized formulations to be purchased. In many cases, a customer will leave with a large number of individual cosmetic items. The individual containers, each with their own box, are bagged and given to the customer. Typically, each container has a three month supply of cosmetics.

The contents of a given container are usually mixed and filled in a manufacturer's facility approximately 6-9 months prior to being distributed to retailers. It typically takes an additional three months to get the containers placed on a shelf in each store, and the products can then sit of the shelf for as long as 2 years until purchased. The products thus must be robust to maintain their effectiveness for such a long time. The long timeline is still undesirable, however, because of its effect on freshness, as well as the large number of individual containers typically purchased by the customer during a single consultation. All of the luxury brands of cosmetic operate in basically the same way and have therefore the same disadvantages.

The present invention is directed toward a significantly different approach to the distribution and sale of high end/luxury cosmetics.

### Summary of the Invention

Accordingly, present invention is defined in the attached claims.

### Brief Description of the Drawings

Figure 1A is an elevational view of a truffle-like cosmetic portion, similar in size and shape to a confectionary truffle.
Figure 1B is a cross sectional view of the cosmetic portion of Figure 1A.
Figure 2 is an elevational view of the truffle portion of Figure 1 covered in a wrapper.
Figure 3 is a schematic view of a plurality of truffle portions arranged in a truffle signature box.
Figure 4A is a perspective view of an appliance for transforming the truffle cosmetic portion of Figure 1 into a formulation which is applied by a user.
Figure 4B is a perspective view of the appliance of Figure 4A in an opened position, displaying the formulation for use.
Figure 5A is a perspective view of a mold plate for producing the truffle portions of Figure 1.
Figure 5B is a cross sectional view showing a first step in the manufacture of the truffle portions.
Figure 5C is a cross sectional view showing a second step in the manufacture of the truffle portions.
Figure 5D is a third step in the manufacture of the truffle portions.
Figure 5E is a fourth step in the manufacture of the truffle portions.

### Best Mode for Carrying Out the Invention

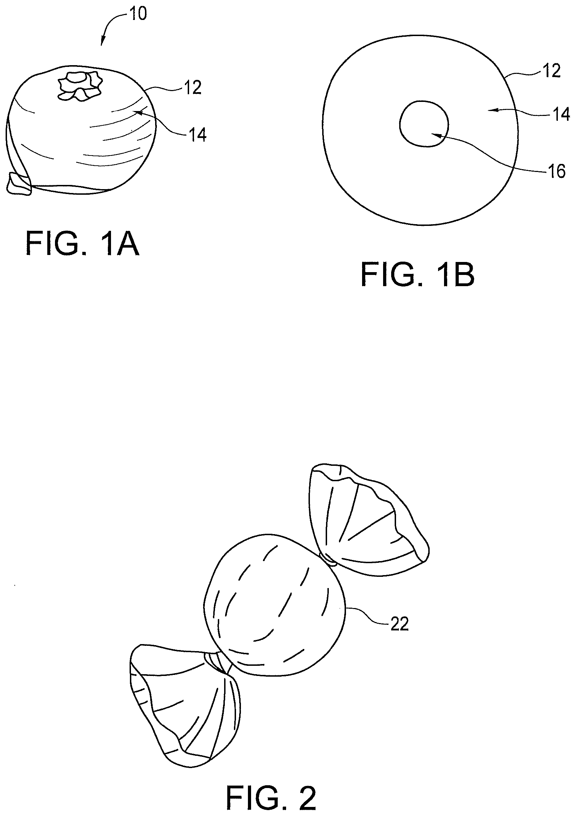

Figures 1A and 1B show the truffle cosmetic portion or member of the present invention. The word truffle used herein means a product portion or member described herein which is configured to have the appearance of a confectionary truffle. It is, however, not a confectionary product. The truffle shown generally at 10 includes a protective coat outer layer 12, an inner volume of cosmetic 14 and, in some embodiments, a central capsule 16, such as typically found in the nutrition industry, also with a protective coating. The outer protective coat acts as a water barrier, preventing water from escaping the cosmetic 14 and water from entering the truffle portion. The protective coat has a melting point typically in the range of 90-110 degrees Fahrenheit. A typical range of thickness is 0.5mm-5mm, with a volume of 0.5-2.0ml or in some cases 4ml or larger, although these dimensions can vary further. The outer layer is chemically compatible with the cosmetic 16 and the central capsule when one is present. The outer layer 12 comprises various materials including, but not limited to, materials found in enteric coatings, including fatty acids, plant and animal waxes (e.g. paraffin, carnuba wax, beeswax, stearic acid, lanolin, soy wax), shellac and animal fibers, fats (e.g. vegetable fats), resins, oils, starches, among others. Wax is an excellent barrier to water but is relatively brittle in its pure form so that a typical protective coat/outer layer will consist of a combination of wax and coconut oil in a 1:2 or 1:3 ratio. Resin (or similar material) can be mixed with the wax/oil combination to alter properties of the outer layer, typically resulting in a slightly higher melting point. Alternatives to coconut oil include coconut butter, argon Oil and Shea Butter. Still other possibilities include mineral oil and paraffin. In addition to a combination of wax and oils, other materials, including sodium alginate/calcium, agar, agarose, carrageenan, gelatin and hydra gels can be used, all of which are designed to protect the internal cosmetic material 14.

The inner volume of cosmetic material 14 can vary significantly. The cosmetic can be those used to cleanse, hydrate, treat, protect or even color the skin. The formula can be either aqueous or oil based. The cream could be pure Shea Butter (i.e. oil). It could also be a complex, special formula supplied by a contract manufacturer. However, it should melt in the range of 90-110 degrees Fahrenheit and be blendable with the other parts of the truffle including in particular the outer coating. The cosmetic could be various skin creams which treat oily skin, dry skin, acne skin, sensitive skin and normal skin. The function of the cream could be hydrating, or a barrier cream which prevents water loss from the skins' surface, or other treatment creams, including anti-aging creams, calming creams, and nourishing creams.

The center capsule 16 is similar to conventional capsules. This part of the truffle is not essential but is important in particular cases; the truffle can comprise the protective outer layer and the cosmetic alone. The capsule 16 when present will typically be a capsule made of gelatin or other encapsulating materials, including non-animal products such as cellulose, ranging in volume from 10ml - 500ml, containing vitamins or an oil, or other active ingredients or a fragrance. Various beauty oils can be used including, but not limited to, for example, argan oil, mango oil, sandalwood, avocado oil, coconut, grape seed, and evening primrose. Essential oils can also used for skincare, including for instance, rose oil, carrot seed, frankincense, geranium and many others, which are directed toward skin care issues, including acne, aging, scarring, oiliness, dryness or elasticity. The capsule could also include prescription ingredients. Some of the active ingredients could be vitamins A, E, C, BHA beta-hydroxy acid, salicylic acid, AHA alphahydroxy acids, various proteins and peptides, skin darkening or lightening agents and liposomes.

The capsule must not dissolve within the cream center prior to blending. Further, it should not be a solid. It must melt or dissolve when placed in an appliance for blending and heating. Soft gel caps can be used as well as the harder gelatin capsules or even wax capsules. The capsule ingredients are enclosed within the gel cap or similar substance to remain separate from the cream until the truffle is blended. Several capsules can be included in the center of the cosmetic volume 14 of a single truffle. In such an arrangement, a synergy could result when the truffle is blended and heated.

In general summary, the truffle member or portion includes an outer protective coating, typically a wax/oil combination, an inner cosmetic volume, and a capsule containing an active or other ingredient described above. The truffle can be decorated with various components, including mineral or metal elements, in various ways, including various design/initials.

Each truffle cosmetic member can be individually wrapped at 20 to extend shelf life or to increase the attractiveness of the truffle, as illustrated in Figure 2. The wrapper 20 can also have various designs and can be of various materials, including foil and paper, among others. The individuality of the truffle can be embellished by the wrapper.

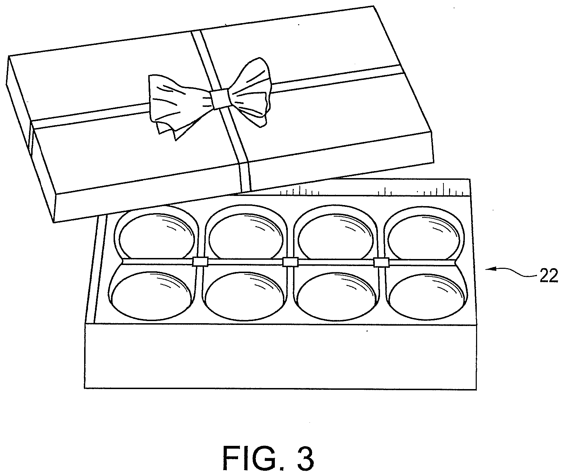

A plurality of individual truffles can be placed in a special truffle box 22 as shown in Figure 3. The truffle box 22 has the appearance of an assortment of chocolate candy truffles, providing a distinctive brand appearance which is substantially different than a plurality of containers, jars, tubes etc. provided by existing luxury brands for its cosmetics. The truffle member concept of the present invention is thus substantially different not only in its structural approach to cosmetics and the conventional cosmetics counter but in the appearance and commercial presentation of the truffle member.

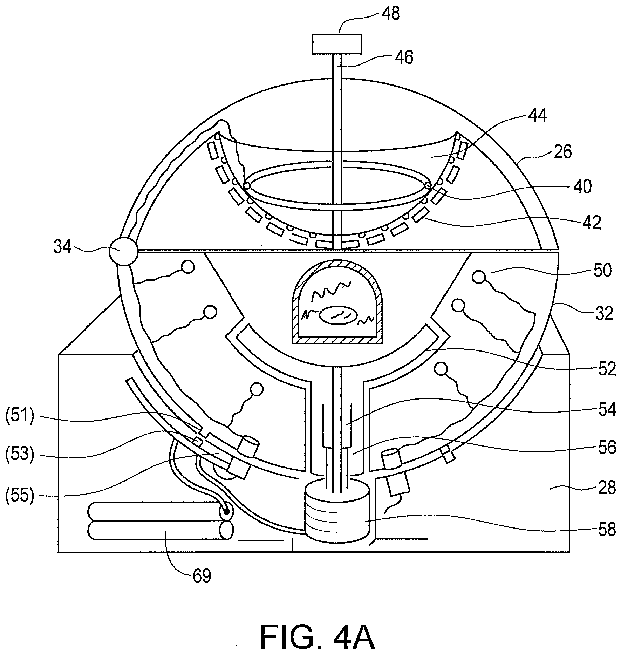

In order for the user to make use of the truffle cosmetic member described above, the truffle must be placed in an appliance, e.g. an amulet container which is designed to hold the truffle and operate on the truffle to make it usable. The amulet is typically spherical and is shown in cross sectional form in figure 4A. The amulet 26 is positioned on a motor platform 28 which can be positioned on a shelf or counter or the like. The appliance can be powered by batteries (direct current) or passively charged. The spherical amulet includes a top portion 30 which can be lifted off a lower portion 32 by a side hinge 34. The top portion in use is lifted up, and the truffle 36 is inserted into the lower portion after the wrapper, if present, is removed from the truffle cosmetic member. The top portion 30 includes a cup-like depending portion 44 which is attached to the inner surface of top portion 30. The depending cup portion 44 includes upper heating elements 40. A mesh grid 42 surrounds the depending portion 44. A plunger 46 extends down through dependent portion 44 with the lower end thereof secured to the mesh grid 42. The upper end of the plunger 46 extends out of the top portion, terminating in a push button element 48.

The lower part includes another heating element 50 and a rotating curved blade 52 which has a mechanical connection 54 which is mated to a drive member 56 from motor 58, with the motor being powered by a battery 69 which can be charged.

In operation, push button 48 is moved downwardly by the user, moving the mesh 42, mashing the truffle against blades 52. In addition to mashing the truffle member, pushing down on push button 48 causes the entire amulet to move downward, where a small projection 51 on the lower surface of the amulet actuates an on/off button 53. This starts a software program on circuit board 55 to actuate blades 52, which are retractable, and provides current to heating elements 40 in the top portion 50 and in the bottom portion of the amulet. The blades operate at a sufficient speed to blend the parts of truffle together, resulting in a creamy formulation. Although the described embodiment uses moving blades, with a revolving or an oscillating movement, other arrangements can be used to produce the blending, including for instance ultrasound or other mechanical means, such as a plunger The depending part 44 in the top portion is in the same shape as the mesh grid and is textured to mate with the mesh grid so that material is pushed out of the mesh in operation as the mesh grid is moved upwardly back against the dependent part 44. The truffle member is blended with heat, to form a conventional, usable formulation.

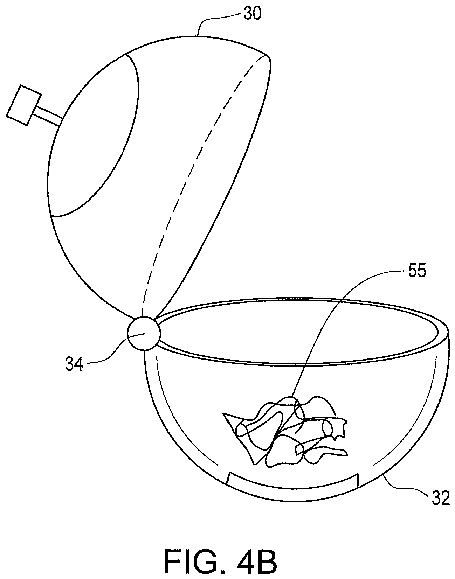

Referring to Figure 4B, the formulation is accessible by lifting the top portion 30 of the amulet off the bottom portion 32 abut hinge 34. The size of the amulet in conjunction with the size of the truffle produces a desired supply of the formulation 55, i.e. a few days (or even one application) to perhaps a month supply. When the formulation is used up, the amulet can be washed to receive a new truffle. While an appliance is typically used to blend and warm the truffle into a formulation, in some cases, when the truffle has a particular composition, the blending and warming of the truffle can be accomplished by hand action alone.

The truffle of Figures 1A and 1B can be manufactured in various ways. One current method is described as follows. It should be understood that the various process steps described below are illustrative only and can be varied, depending upon the desired characteristics and function of the final blended formulation.

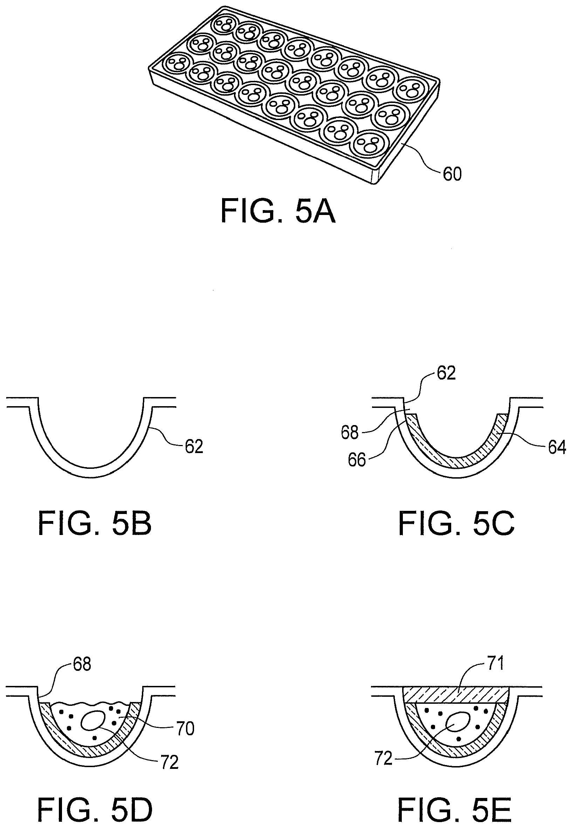

In the present arrangement, the truffle members are processed individually in a mold plate such as 60 shown in Figure 5A. In a double boiler filled with water, the top part of the boiler also has water. The water in the top part is heated to simmering. 50 grams of a wax mixture is added to the upper part and slowly stirred until the wax is melted. The internal gel capsule is dipped into the melted wax, insuring that the capsule is completely coated, then placed on wax paper to cool. In another bowl is added wax, butter and oil (wax/butter/oil mixture at the desired ratio, typically in the range of 1:1:1-1:5:5). In some cases, pure butter (e.g. cocoa butter) could be used or wax butter. This bowl is placed in the heated double boiler and the mixture stirred until it is completely melted. Typically the temperature will depend on the selected ratio, but usually ranges from 90°F to 110°F. This is the outer layer material of the truffle.

The inside of each truffle mold 62 Figure 5B in the mold plate 22 is first coated with a releasing agent such as olive oil. 3 ml of the melted wax/oil mixture is poured into each mold. The mold can be warmed to slow the natural solidification of the wax/oil mixture. Figure 5C shows mold 62 and a wax/oil shell 64. The mold is then placed in a refrigerator to cool for a few minutes. The mold is removed and inverted to remove any remaining liquid mixture. At this point the mold edge 66 has a slight head space 68 which is below the top of the mold. This headspace is filled with additional wax/oil mixture once the other components are added.

A pastry bag or similar container filled with the desired cosmetic 70, such as one of the various possibilities discussed above, is used to fill each shell with approximately 0.5-1.0 ml of cosmetic. This is shown in Figure 5D. The cream should not extend above the mold shell. At this point, one or more wax coated capsules 72 can be inserted into the cosmetic, if desired.

The remaining head space is then filled with liquid wax/oil mixture. The cosmetic is completely covered by the wax/oil mixture, shown at 71 in Figure 5E with the interior cosmetic 70 and the capsule 72.

The mold is then placed in a freezer for approximately 15 minutes to solidify the truffle member. The mold is then removed from the freezer, inverted and the truffle members released.

It should be understood that the manufacturing process disclosed above is one example only of how to make the truffle members.

Decoration of the resulting truffle is referred to briefly above. This can occur at several places during the process of manufacture. Edible gold flakes can be added to the releasing agent, such as olive oil, to give a golden sheen to the entire surface. Once the truffles are released from the mold, gold leaf 74 (Figure 1A) can be added to the surface using a small brush. Decorations can be other elements including mica or other minerals such as silver, mineral make-up powder, even food grade dyes can be used to decorate and embellish the appearance of the truffle members.

As discussed above, the individual truffles are then made available for display, either wrapped or unwrapped, depending on the truffle context. The truffles are typically provided in a display counter at retail, similar to a display used in a high end chocolate shop. A beauty advisor is typically needed to help select different truffles for each customer's skin type and need. Consumers purchase their two week or typically one month supply of each truffle. Generally, a single truffle will be for one day or two weeks or one month, although this could vary. As one example, for anti-aging, the beauty advisor could recommend truffles with Retinol capsules, where each month's supply would be an anti-aging truffle with Retinol treatment capsules. The first month's supply (30 truffles) could include a shea cream cosmetic center while the next month a proprietary cosmetic could be included and the following month a coconut butter cosmetic. The same approach could be used with different fragrances. Further, there may be different truffle products for morning and for at night.

At this point, once the truffle selection has been made, the beauty advisor will place each truffle in a segmented specialty box. The box is wrapped and the consumer carries it away like a chocolate box or a hat box. The size of the box will vary depending on the need or the skin issue of the customer.

First time customers will have to purchase the appliance, i.e. the amulet and the motor mount. The beauty advisor will demonstrate the use of the appliance with a truffle. The truffle is first placed in the amulet. The amulet is then placed on the motor mount. Operating the pushbutton will produce the predetermined mashing, blending, and heating of the truffle. When completed, the customer can remove the amulet. The amulet is then opened, typically revealing a warm, fragrant, fluffy cream that can be applied to the skin directly by the user. Typically, the motor mount will only be needed to blend the truffle one time. However, the amulet can be used to heat the blended formulation for each actual use if desired. The customer can use the appliance at home.

There are several advantages of the present invention over existing luxury cosmetic brands, including fresh preparation, customized for the individual skin and easier to use. The resulting warm formulation is is easy to apply and absorbs better into the skin, providing a novel sensory experience. It should be understood that each truffle member is a single use to avoid spoilage, as can occur with a conventional three month bottle supply of cosmetic. It should also be understood that the size of the truffle can vary depending on the need to create up to a month's supply in the amulet.

As indicated above, the amulet is reusable, easy to wash and prepare for the next truffle. It is possible to have several amulets, depending upon, for instance, whether there is a difference between the night cosmetic and the day cream as well as perhaps more than one amulet for different treatment formulations. All amulets are capable of attaching to the same motor platform.

Accordingly, a new cosmetic system has been described using a cosmetic truffle member, which is individually made and then processed by the user in an amulet appliance to blend and heat the truffle, producing a warm, fragrant cream for application to the user's face, body or hair.

## Claims

1. A method for preparing a skin formulation, the method comprising:
(a) placing a cosmetic article into an appliance configured for blending the cosmetic article, wherein the appliance comprises:
a container for the cosmetic article having a heating assembly and elements for blending the article into the skin formulation; and
a mechanism operable by the user for operating the base member to blend and heat the cosmetic article into the skin formulation;
wherein the placing comprises placing the cosmetic article into the container of the appliance;
wherein the cosmetic article comprises:
a water resistant outer layer having a melting point in the range of 32.2-43.3 degrees Celsius (90-110 degrees Fahrenheit), a thickness of 0.5mm-5mm, and that defines an interior volume;
a cosmetic material contained within the interior volume of the outer layer;
wherein the cosmetic material and the outer layer are configured to blend with one another to produce the skin formulation;
and
(b) heating and blending the cosmetic article in the appliance to form the skin formulation, wherein the blending comprises moving the blending element to blend the cosmetic article within the container, and wherein the heating comprises heating the cosmetic article within the container with the heating element.

2. The method of claim 1, wherein the appliance comprises a motorized base member to which the container is connectable for moving the blending elements.

3. The method of claim 1 or claim 2, wherein the skin formulation is formed entirely from the cosmetic article.

4. The method of claim 1, claim 2, or claim 3, wherein the heating and blending produces the skin formulation in the form of a cream that can be applied to a user's skin directly by the user.

5. The method of any one of claims 1-4, wherein the heating comprises providing electrical current to the heating element.

6. The method of any one of claims 1-5, wherein the blending comprises rotating the blending element to blend the cosmetic article.

7. The method of any one of claims 1-6, wherein the appliance comprises a motor configured to move the blending element, and wherein the blending comprises providing electrical current to the motor to move the blending element.

8. The method of claim 7, wherein the appliance further comprises an on/off button configured to control provision of electrical current to the heating element and the motor, and wherein the method further comprises:
actuating the on/off button to provide electrical current to the motor and the heating element.

9. The method of any one of claims 1-8, wherein the appliance comprises a top portion and a bottom portion that together define the container, wherein the method further comprises:
mashing the cosmetic article against the blending element with the top portion.

10. The method of any one of claims 1-9, further comprising:
washing the container to remove the skin formulation;
placing a second cosmetic article in the appliance; and
heating and blending the second cosmetic article in the appliance to form a new skin formulation.

11. The method of any one of claims 1-10, wherein the outer layer comprises a combination of wax and oil.

12. The method of any one of claims 1-11, wherein the skin formulation comprises retinol.

## Patentansprüche

1. Verfahren zur Herstellung einer Hautformulierung, wobei das Verfahren Folgendes umfasst:
(a) Platzieren eines Kosmetikartikels in einem Gerät, das konfiguriert ist, um den Kosmetikartikel zu mischen, wobei das Gerät Folgendes umfasst:
einen Behälter für den Kosmetikartikel mit einer Heizanordnung und Elementen zum Mischen des Artikels in die Hautformulierung; und
einen Mechanismus, der durch den Benutzer bedienbar ist, um das Basiselement zu bedienen, um den Kosmetikartikel in die Hautformulierung zu mischen und zu erhitzen;
wobei das Platzieren Platzieren des Kosmetikartikels in dem Behälter des Geräts umfasst;
wobei der Kosmetikartikel Folgendes umfasst:
eine wasserbeständige Außenschicht mit einem Schmelzpunkt in dem Bereich von 32,2-43,3 Grad Celsius (90-110 Grad Fahrenheit), einer Dicke von 0,5-5 mm, und die ein Innenvolumen definiert;
ein Kosmetikmaterial, das innerhalb des Innenvolumens der Außenschicht enthalten ist;
wobei das Kosmetikmaterial und die Außenschicht konfiguriert sind, um sich miteinander zu mischen, um die Hautformulierung zu produzieren; und
(b) Erhitzen und Mischen des Kosmetikartikels in dem Gerät, um die Hautformulierung zu bilden, wobei das Mischen Bewegen des Mischelements umfasst, um den Kosmetikartikel innerhalb des Behälters zu mischen, und wobei das Erhitzen Erhitzen des Kosmetikartikels innerhalb des Behälters mit dem Heizelement umfasst.

2. Verfahren nach Anspruch 1, wobei das Gerät ein motorisiertes Basiselement umfasst, mit dem der Behälter verbindbar ist, um die Mischelemente zu bewegen.

3. Verfahren nach Anspruch 1 oder Anspruch 2, wobei die Hautformulierung vollständig aus dem Kosmetikartikel gebildet wird.

4. Verfahren nach Anspruch 1, Anspruch 2 oder Anspruch 3, wobei das Erhitzen und Mischen die Hautformulierung in der Form einer Creme produziert, die auf die Haut eines Benutzers direkt durch den Benutzer aufgetragen werden kann.

5. Verfahren nach einem der Ansprüche 1-4, wobei das Erhitzen Bereitstellen von elektrischem Strom an das Heizelement umfasst.

6. Verfahren nach einem der Ansprüche 1-5, wobei das Mischen Drehen des Mischelements umfasst, um den Kosmetikartikel zu mischen.

7. Verfahren nach einem der Ansprüche 1-6, wobei das Gerät einen Motor umfasst, der konfiguriert ist, um das Mischelement zu bewegen, und wobei das Mischen Bereitstellen von elektrischem Strom an den Motor umfasst, um das Mischelement zu bewegen.

8. Verfahren nach Anspruch 7, wobei das Gerät ferner einen Ein-/Aus-Knopf umfasst, der konfiguriert ist, um Bereitstellung von elektrischem Strom an das Heizelement und den Motor zu steuern, und wobei das Verfahren ferner Folgendes umfasst:
Betätigen des Ein-/Aus-Knopfes, um elektrischen Strom an den Motor und das Heizelement bereitzustellen.

9. Verfahren nach einem der Ansprüche 1-8, wobei das Gerät einen oberen Teil und einen unteren Teil umfasst, die zusammen den Behälter definieren, wobei das Verfahren ferner Folgendes umfasst:
Zerdrücken des Kosmetikartikels gegen das Mischelement mit dem oberen Teil.

10. Verfahren nach einem der Ansprüche 1-9, ferner umfassend:
Waschen des Behälters, um die Hautformulierung zu entfernen;
Platzieren eines zweiten Kosmetikartikels in dem Gerät; und
Erhitzen und Mischen des zweiten Kosmetikartikels in dem Gerät, um eine neue Hautformulierung zu bilden.

11. Verfahren nach einem der Ansprüche 1-10, wobei die Außenschicht eine Kombination aus Wachs und Öl umfasst.

12. Verfahren nach einem der Ansprüche 1-11, wobei die Hautformulierung Retinol umfasst.

## Revendications

1. Procédé permettant la préparation d'une formulation cutanée, le procédé comprenant :
(a) le placement d'un article cosmétique dans un appareil conçu pour mélanger l'article cosmétique, ledit appareil comprenant :
un récipient pour l'article cosmétique possédant un ensemble chauffant et des éléments destinés à mélanger l'article en la formulation cutanée ; et
un mécanisme actionnable par l'utilisateur pour actionner l'élément de base pour mélanger et chauffer l'article cosmétique en la formulation cutanée ;
ledit placement comprenant le placement de l'article cosmétique dans le récipient de l'appareil ;
ledit article cosmétique comprenant :
une couche externe résistante à l'eau possédant un point de fusion dans la plage de 32,2 à 43,3 degrés Celsius (90 à 110 degrés Fahrenheit), une épaisseur de 0,5 mm-5 mm, et qui définit un volume intérieur ;
un matériau cosmétique contenu dans le volume intérieur de la couche externe ;
ledit matériau cosmétique et ladite couche externe étant conçus pour se mélanger l'un à l'autre pour produire la formulation cutanée ; et
(b) le chauffage et le mélange de l'article cosmétique dans l'appareil pour former la formulation cutanée, ledit mélange comprenant le déplacement de l'élément de mélange pour mélanger l'article cosmétique à l'intérieur du récipient, et ledit chauffage comprenant le chauffage de l'article cosmétique à l'intérieur du récipient avec l'élément chauffant.

2. Procédé de la revendication 1, ledit appareil comprenant un élément de base motorisé auquel le récipient peut être relié pour déplacer les éléments de mélange.

3. Procédé de la revendication 1 ou la revendication 2, ladite formulation cutanée étant entièrement formée à partir de l'article cosmétique.

4. Procédé de la revendication 1, la revendication 2 ou la revendication 3, ledit chauffage et ledit mélange produisant la formulation cutanée sous la forme d'une crème qui peut être appliquée sur la peau d'un utilisateur directement par l'utilisateur.

5. Procédé de l'une quelconque des revendications 1-4, ledit chauffage comprenant la fourniture d'un courant électrique à l'élément chauffant.

6. Procédé de l'une quelconque des revendications 1-5, ledit mélange comprenant la rotation de l'élément de mélange pour mélanger l'article cosmétique.

7. Procédé de l'une quelconque des revendications 1-6, ledit appareil comprenant un moteur conçu pour déplacer l'élément de mélange, et ledit mélange comprenant la fourniture d'un courant électrique au moteur pour déplacer l'élément de mélange.

8. Procédé de la revendication 7, ledit appareil comprenant en outre un bouton marche/arrêt conçu pour commander la fourniture de courant électrique à l'élément chauffant et au moteur, et ledit procédé comprenant en outre :
l'actionnement du bouton marche/arrêt pour fournir du courant électrique au moteur et à l'élément chauffant.

9. Procédé de l'une quelconque des revendications 1-8, ledit appareil comprenant une partie supérieure et une partie inférieure qui définissent ensemble le récipient, ledit procédé comprenant en outre :
l'écrasement de l'article cosmétique contre l'élément de mélange avec la partie supérieure.

10. Procédé de l'une quelconque des revendications 1-9, comprenant en outre :
le lavage du récipient pour éliminer la formulation cutanée ;
le placement d'un second article cosmétique dans l'appareil ; et
le chauffage et le mélange du second article cosmétique dans l'appareil pour former une nouvelle formulation cutanée.

11. Procédé de l'une quelconque des revendications 1-10, ladite couche externe comprenant une combinaison de cire et d'huile.

12. Procédé de l'une quelconque des revendications 1-11, ladite formulation cutanée comprenant du rétinol.
